# EUROPEAN PATENT APPLICATION

(11) **EP 3 815 726 A1**
(43) Date of publication of application: **05.05.2021**
(21) Application number: 19825429.4
(22) Date of filing: 07.06.2019
(51) Int. Cl.: A61M 5/142

(54) **SYRINGE INFUSION PUMP**

(30) Priority: 29.06.2018 RU 2018123827
(71) Applicant: Limited Liability Company "Medplant", Moscow, 109316 (RU)
(72) Inventor: MAMDZHYAN, Garegin Grigorevich, Moscow, 107258 (RU); OSIPOV, Aleksei Yurevich, Moscow, Zelenograd, 124460 (RU); SMELOV, Vladimir Sergeevich, Moscow, 121601 (RU)
(74) Representative: Cabinet Chaillot
(86) International application number: PCT/RU2019/000408
(87) International publication number: WO 2020/005107

(57) **Abstract**

The invention belongs to the sphere of medical equipment. The medicine dispenser syringe comprises the body with an affixed syringe equipped with a plunger, a plunger pusher driven by a motor, as well as a power supply unit and a control unit consisting of a processor, a control panel and a display for showing infusion parameters. According to the invention, the dispenser syringe is additionally equipped with a pressing rod located perpendicular to the syringe. There is a movable clamp at the upper end of the rod, while at the lower end there is a slider connected to the movable rod of the potentiometer, the signal from which is transmitted to the control unit (via the databus line). The plunger pusher of the syringe is affixed onto the threaded shaft. A motor revolution counter is installed on the threaded shaft; it includes a disk diaphragm with radial slits rotating on it. The diaphragm is located between the led and the phototransistor - to control the passage of light flux. The disk diaphragm is connected to the motor via an adapter sleeve to enable its rotation.

The technical result of the invention is to increase the consumer properties of the dispenser syringe, which will allow for its use in emergency situations, including on-site conditions, due to its minimum dimensions, weight, low price and full automation of the infusion process.

## Description

The invention relates to medical equipment, namely to dispenser syringes that serve for the administration of various medications with high dosing accuracy. Dispenser syringes are intended for patients with various diseases (oncology, cardiology, diabetes, etc.) and are to be used by various medical institutions.

The featured medical infusion system includes a pumping mechanism with a toothed coupling, a cassette having a cylindrical cartridge with a reservoir for the medication (the outer surface of the cartridge having a grooved or threaded surface), as well as a motor and an application for a linear sensor. The motor causes bidirectional pumping so that the samples could be presented in the sensor area (US20060079831, cl. A61M1/00, A61M31/00, A61M37/00, published on 13.04.2006).

The disadvantage of the featured device is the lack of automatic determination of the syringe diameter, and since the diameter of the same-type syringes may significantly vary, there is a possibility of incorrect input of the speed and volume of the infusion when using such an infusion system.

The featured micro-pump with a display of information about patients and medications contains an injector clamping mechanism mounted on the lower part of the front panel of the micro-injection pump, a nozzle pushing mechanism, an injection flow rate control panel located on the front panel of the pump with a micro-supercharger, and a locking mechanism located on the back panel of the micro-injection pump. The microinjection pump is equipped with an internal information control unit and a keyboard input device, as well as a display, which are connected to the information control unit. The keyboard input device and the information display device are located on the microinjection pump. During the use of the device, the keyboard is used to enter patient information and other useful data (such as the liquid medicine being administered), which is shown on the display. This not only ensures the convenient operation of the device, but is also quite handy for the observation; besides, it is also possible to re-enter patient information during the next use (CN201431672, cl. A61M5/142, G06F3/023, G09F9/00, published on 2010-03-31).

The lack of automatic determination of the syringe diameter does not ensure the accuracy of the infusion and the safety of medicine administration.

The prototype of the invention is a medical dispenser syringe, its body having an affixed syringe (equipped with a plunger and a motor-driven plunger pusher). The body also has a power supply unit and a control unit consisting of a processor, a control panel and a display for showing infusion parameters. The display includes operator-activated information input mechanisms (e.g. keyboard, touch screen functions, switches, microphone, etc.). The presence of a communication port makes it possible to connect the dispenser syringe to additional equipment, such as laptops, pocket programming devices and/or network equipment (US20160331895, cl. A61M5/1452; A61M5/16854; A61M5/16859; A61M5/5086; A61M2005/16863; A61M2205/3569; A61M2205/505; A61M2205/52, published on 2016-11-17).

This device, however, presupposes only manual determination of the syringe equipment characteristics necessary for the correct input with the required infusion rate.

The technical challenge of the invention is the creation of a portable and compact medicine dispenser syringe with automatic high-precision determination of syringe diameter and high-accuracy control of the infusion rate (at least 2%), as well as of a convenient and simple device that will come in handy when working in emergency medical care, especially on-site.

The technical result of the invention is to increase the consumer properties of the dispenser syringe, which will allow for its use in emergency situations, including on-site conditions, due to its minimum dimensions, weight, low price and full automation of the infusion process.

The described technical challenge is managed by the fact that the body of the medicine dispenser syringe has an affixed syringe with a plunger, a plunger pusher driven by a motor, as well as a power supply unit and a control unit consisting of a processor, a control panel and a display for showing infusion parameters. According to the invention, the dispenser syringe is additionally equipped with a pressing rod located perpendicular to the syringe, with a movable clamp installed at the upper end of the rod, and a slider installed at the lower end; the slider is connected to the movable rod of the potentiometer that emits the signal transmitted to the control unit through the databus line. The plunger pusher of the syringe is affixed onto the threaded shaft; the shaft also has a motor revolution counter installed, together with a disk diaphragm with radial slits that rotates on the threaded shaft and is located between the led and the phototransistor - to control the passage of the light flux.

The disk diaphragm is connected to the motor via an adapter sleeve to enable its rotation. A DC motor of continuous rotation is used as the motor.

The body is equipped with a rear protective cover and fasteners.

The overall dimensions of the dispenser are 165 x 74 x 29 mm; its weight constitutes 0.3 kg.

The presence of the pressing rod equipped, at its lower end, with a slider connected to the movable rod of the potentiometer (it emits the signal that is transmitted, via the databus line, to the processor) can accurately determine the characteristics of the syringe according to the magnitude of the signal from the potentiometer caused by the displacement of the slider on the clamping rod. Since syringes of even the same size have a noticeable variation in diameter, not to mention different types (from 5 to 20 ml), it is necessary to measure the diameter of a syringe quite accurately (at least ±2 %) in order to correctly enter the infusion rate of the medicine.

The installation of a revolution counter (consisting of a led, a disk diaphragm with radial slits and a phototransistor to control the passage of the light flux) on the threaded shaft makes it possible to set a certain value for the infusion rate. This greatly simplifies the operation of the dispenser syringe (since only one parameter needs to be entered - the infusion rate) and makes it possible to use the syringe dispenser in emergency medical care, in particular on-site.

The use of a DC motor of continuous rotation will significantly reduce the cost of the device as a whole by using a discrete mode of activating the motor with the duty cycle that determines the average speed of rotation of the threaded shaft.

The presence of a rear protective cover on the dispenser body makes it possible to place it on the patient's bed or gurney without the risk of tissue getting into the chassis and stopping the motor.

The presence of fasteners on the dispenser body ensures that it is attached to flat splints in ambulances, to the belts used to affix devices onto the patient's arm, or to round splints (on gurneys, beds, ambulances), which makes the device convenient when providing emergency medical services.

The dispenser syringe makes it possible to simplify the operation of the device as much as possible: due to the fact that only one parameter needs to be entered - the flow rate of the medicine. In combination with its small size, weight and low price, the device is a very convenient tool when working in emergency medical conditions, especially on-site. You can use any single-use syringes with the filling volume of 5 - 20 ml.

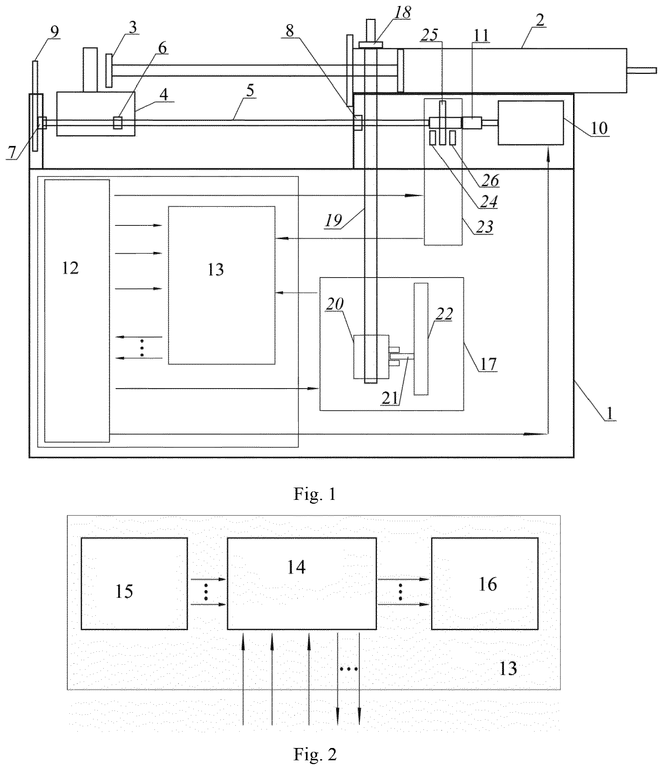

The invention is explained by drawings, where Fig. 1 shows a block diagram of the medicine dispenser syringe, and Fig. 2 shows a block diagram of the control unit.

The medicine dispenser syringe consists of Body 1, on which Syringe 2 is installed. Plunger 3 of Syringe 2 rests against Pusher 4, which is affixed onto Threaded Shaft 5 by means of Threaded Coupling 6. Threaded Shaft 5 is affixed onto Body 1 by means of End Sleeve 7, Lead-Through Sleeve 8 and Latch 9, which makes it possible to remove Shaft 5 without disassembling Body 1 of the device. Threaded Shaft 5 is driven by DC Motor of Continuous Rotation 10 through Adapter Sleeve 11. The board of Body 1 of the dispenser syringe has the following elements installed: Power Supply Unit 12 and Control Unit 13 consisting of Processor 14, Control Panel 15 and Display 16 for showing infusion parameters. The board also has Syringe Diameter Measurement Unit 17. Syringe 2 is affixed onto Body 1 by means of Clamp 18 installed at the upper end of Pressing Rod 19, perpendicular to Syringe 2; at the lower end of Pressing Rod 19 there is Slider 20 connected to Movable Rod 21 of Potentiometer 22 (that transmits its signal, via the databus line, to Processor 14 of Control Unit 13). Besides, Threaded Shaft 5 has Revolution Counter 23 located in Body 1. Counter 23 consists of Led 24, Diaphragm 25 (in the form of a disk with radial slits), and Phototransistor 26 - for controlling the passage of the light flux. Diaphragm 25 is rotated by Motor 10 through Adapter Sleeve 11.

The medicine dispenser syringe works as follows.

Syringe 2, filled with the medicine, is installed onto Body 1. Pusher 4 is moved all the way to Plunger 3 of Syringe 2. The device is switched on; the desired mode and its parameters are set; the Start/Stop button is pressed (not shown in Fig.). Processor 14 of Control Unit 13 receives the start signal from Control Panel 15 and transmits the start command to AC Motor 10 via Power Supply Unit 12. Use Motor 10 with an output idle speed of 125 rpm at 3 V voltage. Motor 10 starts to rotate Threaded Shaft 5 through Adapter Sleeve 11 and Diaphragm 25, and Shaft 5 puts Pusher 4 in motion. Pusher 4 begins to move and push Plunger 3 of Syringe 2. The force on Pusher 4 is controlled by the amount of current through Motor 10. The value of the current is measured constantly and automatically. As soon as the current exceeds a certain limit, protection is activated - Processor 14 stops Motor 10. The limit value depends on the type of syringe and the voltage at which Motor 10 operates.

The speed of movement of Pusher 4 and, respectively, Plunger 3 of Syringe 2 is set using Revolution Counter 22. Diaphragm 25 has 8 radial slits (not shown in Fig.); when it rotates, the light flux going from Led 24 to Phototransistor 26 is modeled by moving the slit to the line connecting Led 24 and Phototransistor 26. Light transmission is controlled by Photoresistor 26. A signal appears on Phototransistor 26, which is transmitted onto Processor 14. After the flash pulse, Processor 14 commands Motor 10 to stop, a pause is maintained, and then the motor is activated again, until the next flash pulse through the slits in Diaphragm 25. The rotation speed of Motor 10 is consistent and depends only on the load and voltage. And the average speed of Motor 10 changes and is determined by the duration of the pause. This mode of operation of Motor 10 makes it possible to use cheaper DC motors of continuous rotation with an output idle speed of 125 rpm at a voltage of 3V, rather than more expensive stepper motors. The pause duration is set to the average speed of Motor 10. To convert this rotation speed to the infusion speed, the diameter of the syringe must be determined. The diameter of the syringe is measured using Block 17 by moving Clamp 18 and Slider 20 (affixed to Rod 19) up and down Pressing Rod 19; this prompts the movement of Movable Rod 21, which is part of Potentiometer 22. Potentiometer 22 outputs a signal to Control Panel 15; the signal corresponds to the position of Clamp 18, and therefore to the diameter of the syringe. Processor 14 of Control Panel 15 uses this signal to convert the infusion rate, measured in ml/hour, to the average speed of rotation of Threaded Shaft 5, measured in rpm. The results are output by Processor 14 (via the databus line) to Display 16 for the entered infusion parameters.

At infusion rates from 50 ml/hour to 120 ml/hour, the motor voltage is 2.5 V and all 6 radial slots are counted for 1 revolution (the slots are counted on the next but one principle). In this case, the discreteness is approximately 0.035 ml on a 20 ml syringe. At infusion speeds of more than 120 ml/hour (up to 600 ml/hour), the motor voltage is 2.8 V and the radial slots are counted on the next but two principle, i.e. 4 per revolution. In this case, the discreteness is approximately 0.053 ml on a 20 ml syringe. On syringes of smaller diameter, the discreteness will be less (in proportion to the diameter).

The proposed dispenser syringe has a cardiopulmonary resuscitation (CPR) mode, which is necessary to implement the standard for CPR introduced by the Ministry of Health of the Russian Federation. In this mode, the dispenser is able to squeeze out several doses of 5 ml (or 3 ml) at maximum speed at intervals of 4 minutes and 5 seconds.

The device is equipped with sound and light alarms:
- emergency situations (when occlusion limit is exceeded (increased pressure on the syringe plunger; when the pusher reaches the device cover or stationary (firmly back) plunger of the syringe; when lowering the pressing part during the operation (loss of syringe);
- the process of infusion;
- network availability and battery charging/lack of power.

The proposed device is reliable and easy to operate, it is adapted to be used with syringes of various volumes and purposes, with the possibility of establishing the infusion rate with an accuracy of ±2%. This function is currently available only for stationary dispensers. Besides, this medicine dispenser syringe is a compact and lightweight device (the weight of the dispenser with a battery is 300 g only, in contrast to other known dispensers, the smallest of which weighs 1.5 kg). The presence of a rear protective cover makes it possible to place the device on the patient's bed or gurney without the risk of tissue getting into the chassis and stopping the motor. Besides, the dispenser syringe can be attached to flat splints in ambulances, to the belts used to affix devices onto the patient's arm, or to round splints (on gurneys, beds, ambulances).

Prototypes of the dispenser syringe have been already made, and their mass production is being prepared.

## Claims

1. Medicine dispenser syringe, consisting of a body with a syringe affixed onto it (together with a plunger and a plunger pusher driven by a motor), as well as of a power supply unit and a control unit consisting of a processor, an operation panel and a display showing the parameters of the infusion, ***characterized in that*** it is also equipped with a clamping rod arranged perpendicularly to the syringe; at the upper end of the rod there is a movable clamp, while at the lower end there is a slider connected to the movable rod of the potentiometer (that transfers it signal, via the databus line, to the control unit); the syringe plunger pusher is mounted on the threaded shaft that also has a motor revolution counter installed - the counter activates the disk diaphragm with radial slits that rotates on the threaded shaft and is located between the led and the phototransistor - to control the passage of luminous flux.

2. Dispenser syringe according to claim 1, **characterized in that** the disk diaphragm is connected to the motor through an adapter sleeve to ensure its rotation.

3. Dispenser syringe, according to claim 1, **characterized in that** a DC motor of continuous rotation is used as the motor.

4. Dispenser syringe, according to claim 1, **characterized in that** the body is equipped with a rear protective cover and fasteners.

5. Dispenser syringe, according to claim 1, **characterized in that** the overall dimensions of the dispenser are 165 x 74 x 29 mm and its weight is 0.3 kg.
